# EUROPEAN PATENT SPECIFICATION

(11) **EP 0 320 815 B1**
(45) Date of publication and mention of the grant of the patent: **02.03.1994**
(21) Application number: 88120599.1
(22) Date of filing: 09.12.1988
(51) Int. Cl.: A61M 1/36

(54) **Integrated unit for extracorporeal blood circuits**
Integrierte Behandlungseinheit für extrakorporalen Blutkreislauf
Cellule intégrée pour circuit extracorporel

(30) Priority: 15.12.1987 IT 2301187
(43) Date of publication of application: 21.06.1989
(73) Proprietor: DIDECO S.p.A., I-41037 Mirandola (province of Modena) (IT)
(72) Inventor: Panzani, Ivo, I-41037 Mirandola (Modena) (IT); Ghelli, Nicola, I-40018 San Pietro in Casale (Bologna) (IT); Vescovini, Pietro, I-41056 Medolla (Modena) (IT)
(74) Representative: Modiano, Guido, Dr.-Ing.

(56) References cited:
- EP-A- 0 089 748
- EP-A- 0 114 732
- EP-A- 0 190 020
- US-A- 4 131 431
- US-A- 4 490 331
- US-A- 4 698 207

## Description

The invention relates to an integrated unit for extracorporeal blood circuits.

It is known that during some surgical operations it is necessary to provide an extracorporeal blood circulation in a circuit which comprises a plurality of per se known devices connected by means of connecting lines: one of these devices is the so-called venous tank which collects blood coming from the patient, and another device is the so-called cardiotome which acts as blood storage tank and which normally receives recovery blood from the operating field.

In extracorporeal circulation blood must, according to the requirements, give off or receive heat, and thus a heat exchanger is provided for this purpose, in which blood, while passing through it exchanges heat with a fluid which is normally water; it is furthermore necessary to oxygenate blood, and thus an oxygenating device is provided in extracorporeal circuits.

In known extracorporeal circuits all these devices may be independent from one another, with an easily imaginable confusion of connecting lines, or they may be at least partially integrated, but not in an optimum manner: this leads to difficulties in installation, both due to the complication of the connections to be provided and to the difficulties in the placement of all the devices in a space proximate to the operating field which is normally limited; moreover, no adequately safe provisions are taken to prevent air from being sent into the patient.

In this regard, EP-A-0 190 020 shows a unitary venous return reservoir with a cardiotomy filter including in combination the features as set forth in the precharacterizing portion of the appended claim 1. Furthermore, US-A-4 490 331 shows together with a combination cardiotomy reservoir and venous blood reservoir, an oxygenator and heat exchanger thereby to form a unitary system. In addition, US-A-4 131 431 shows a blood shut off valve having a ball member which floats in the blood reservoir and an outlet valve seat against which the ball member rests in the absence of blood.

The aim of the present invention is to provide an integrated unit for extracorporeal blood circuits having maximum simplicity, so as to facilitate all the operations related to the installation and the control of correct operation, and provides greater safety for the patient.

The proposed aim is achieved by an integrated unit for use in extracorporeal blood circuits, according to the invention, as defined in the appended claim 1.

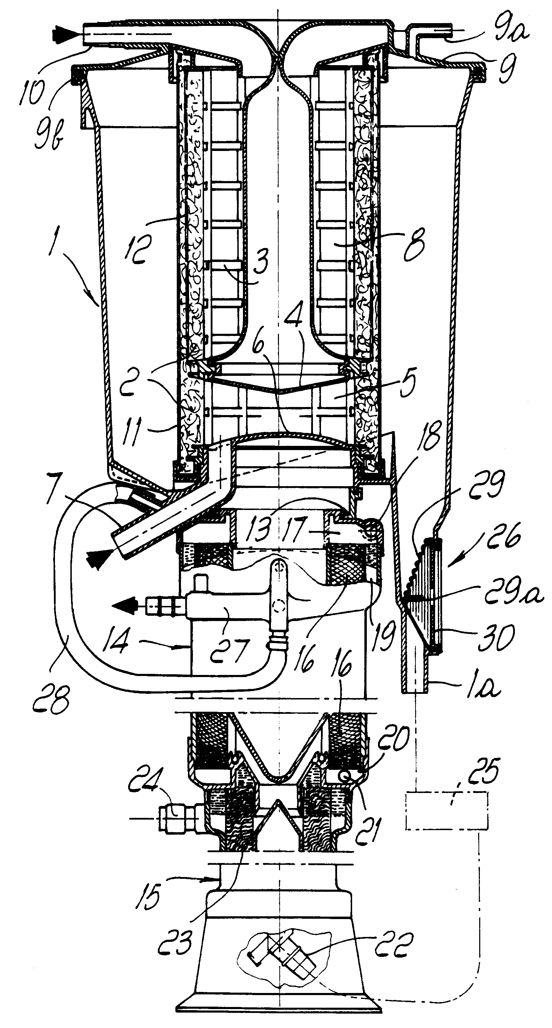

Further characteristics and advantages will become apparent from the description of a preferred, but not exclusive, embodiment of the invention, illustrated only by way of non-limitative example in the accompanying drawing, wherein the only figure is a longitudinal cross section view of the invention with the mechanical safety device at the connection for the outflow of blood from the tank in the position of closure of said connection.

With reference to the above described figure, the reference numeral 1 generally indicates the tank inside which the defoaming material 2 is provided, supported by the supporting structure 3, arranged in the shape of a cylindrical wall so as to delimit in its interior a portion of space which is divided by the partition 4 into two parts.

By means of the inlet connection 7, the blood coming from the patient arrives at the lower part 5, downwardly delimited by the partition 6, while, by means of the connection 10 which extends from the cover 9 of the tank, blood coming from the operating field arrives at the upper part 8, which is upwardly delimited by a portion of said cover.

The filter 11 completely embraces the defoaming material 2 and is sufficient for treating the blood coming from the patient; for treating the blood coming from the operating field the additional filter 12 is provided. After passing through the defoaming material 2 and being appropriately filtered, the blood gathers in the annular capacity delimited by the wall of the tank 1, which is thus capable of simultaneously performing the functions which in known extracorporeal circuits are performed respectively by the venous tank and by the cardiotome.

The described tank 1 is connected by a per se known quick connection 13 to a monolithic structure upwardly comprising the oxygenator 14 and downwardly comprising the heat exchanger 15, both per se known.

Of the oxygenator 14, in any case, the figure illustrates the bundle of capillaries in microporous membrane 16 sunk at the ends into rings of polyurethane resin, termed "potting"; the upper potting determines the annular oxygen compartment 17 with the duct for the inflow of said oxygen 18 and the annular blood compartment 19, while the lower potting determines the oxygen compartment 20 with the duct 21 for the outflow thereof.

Of the heat exchanger 15, the connection 22 for the inflow of blood and the small laminations 23 constituting a wall for the heat exchange between blood and water, which respectively enters and exits by means of connections 24, are visible.

The blood is then drawn, under the action of a pump 25, from the tank 1 by means of the connection 1a controlled by the mechanical safety device generally indicated by 26 which will be described in detail hereinafter, and is sent to the inlet connection 22 of the heat exchanger, after passing therethrough it exits directly into the oxygenator 14, strikes the outside of the capillaries of the bundle 16 and enters the compartment 19 to exit by means of the connection 27 and return to the patient.

The bypass line 28 directly connects the inlet connection 7 to the tank 1 and the outlet connection 27 from the oxygenator 14, and performs the function described hereinafter.

The tank 1 contains blood at atmospheric pressure, being connected to the atmosphere by means of the connector 9a, and clearly it must never empty during operation since in this case the pump 25 would aspirate air which, if sent into the patient, may have lethal consequences. In order to prevent this occurrence, there may be provided, outside the tank 1, per se known level controller sensors, which stop the operation of the pump 25 and turn on an alarm signal upon being near to reach the danger condition, but the real safety against the emptying of the tank is offered by the abovementioned mechanical device 26.

Said device comprises the elastic membrane 29 fixed to the structure of the tank 1, which in normal operating conditions is kept pressed against the small cover 30 by the hydrostatic load of the overlying blood contained in the tank. Therefore, in such a configuration, the connection 1a is left open by the membrane 29 allowing the passage of blood, but the membrane 29 moves to occlude said connection, assuming the configuration illustrated in the figure, when said hydrostatic load reaches a value corresponding to a minimum level of blood in the tank; this way it is impossible to empty the tank.

From the occlusion position, the conditions of normal operation are restored by returning the membrane 29 to the configuration which determines the opening of the connection 1a by manual actuation on the part of an operator on the grip tab 29a, which is gripped and pulled for example by means of forceps inserted in the central hole of the small cover 30.

The mechanical safety device 26 can be provided as an autonomous element, and in this case the elastic membrane 29 and the cover 30 are associated with a structure adapted to be connected to the tank 1.

The intervention of the described safety device, in case the level control sensors are not activated, can determine the release of gas into the blood contained in the aspiration line which connects the temporarily occluded connection 1a to the pump 25, since said blood is placed in depression by the action of said pump.

This blood cannot be reinfused to the patient, and thus, by means of forceps, the line connected to the outflow connection 27 is occluded, and said blood is recycled in the device by means of the bypass 28, removing the means, constituted for example by forceps, which normally occlude the same in normal operating conditions.

The bypass 28, besides performing the described function, also serves to create a recirculation of blood to the oxygenator 14, which cannot remain full of liquid with zero flow, in case of lack of arrival of blood from the patient.

Finally it must be noted that the cover 9 is associated with the tank 1 so as to be rotatable, this way the connection 10 can be orientated as desired, and the sealing gasket 9b is provided in case said tank is employed for a different use from its typical one described up to now, consisting in the post-operative recovery of blood from the drainages; for this use the tank 1 must be placed in depression, and consequently the gasket is necessary and so is a dimensioning of the tank which makes it capable of withstanding the stresses which derive from said depression.

From what has been described the advantages of the invention are apparent, and consist most of all in the drastic reduction of connecting lines with respect to those present in extracorporeal circuits of the prior art: this leads to considerable simplifications in installation, due both to the speed in providing the connections and to the easy arrangement of the circuit determined by the reduction of bulk.

Advantages are observed in the invention in terms of operation control thereof, there having been provided an open tank, as safe as a closed tank, as far as the possibility of sending air into the patient is concerned.

The described invention is susceptible to numerous modifications and variations, all of which are within the scope of the inventive concept; thus, for example, the heat exchanger can be connected directly to the tank, the presence of a pump being provided only at the outlet from the integrated unit according to the invention, or inserted at the inlet of the oxygenator.

In the practical embodiment of the invention, all the details may be replaced with other technically equivalent elements; moreover, the materials employed, as well as the shapes and dimensions, may be any according to the requirements.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. An integrated unit for use in an extracorporeal blood circuit, comprising:
a tank (1) internally provided with a wall of defoaming material (2) including at least one filter (11,12);
a inlet connection (7) for blood coming from a patient and an inlet connection (10) for blood coming from an operating field, said connections (7 and 10) being arranged for passing the blood coming therefrom through said defoaming material (2) and filter (11,12) after which the blood is held in said tank (1) and thereby said tank simultaneously acts both as cardiotome and venous tank; and
an outlet connection (1a) arranged at the bottom of said tank for outflow of the blood contained therein,
the integrated unit being characterized in that it further comprises:
a heat exchanger (15) and an oxygenator (14) which are both connected monolithically to said tank (1) and which are adapted to be traversed by outflowing blood from said outlet connection (1a) of said tank (1); and
a mechanical safety device (26) arranged at said outlet connection (1a) of said tank for determining the closure of said outlet connection upon any emptying of the tank,
said mechanical safety device comprising an elastic membrane (29) adapted to be kept, by the hydrostatic load of the overlying blood, in such a configuration as to keep open outlet connection and so as to move to occlude the same when said load reaches a value corresponding to a minimum level of the blood in the tank.

2. Unit according to claim 1, characterized in that it further comprises a blood circulation pump (25) whose aspiration line is connected to said outlet connection (1a) and whose delivery is connected to a blood inflow connection (22) of said heat exchanger (15), said heat exchanger having an outlet connected to an inlet of the oxygenator (14) from which the blood returns to the patient.

3. Unit according to claim 1, characterized in that said outlet connection (1a) of said tank (1) is directly connected to an inlet (22) of said heat exchanger (15), the unit further comprising, arranged at an inlet of said oxygenator (14), a pump adapted to determine the circulation of the blood and connected, at its aspiration, to an outlet of said heat exchanger (15).

4. Unit according to one or more of the preceding claims, characterized in that said tank (1) is arranged in a position overlying a monolithic structure upwardly comprising said oxygenator (14) and downwardly comprising said heat exchanger (15), and is connected thereto by means of a quick-connection (13).

5. Unit according to one or more of the preceding claims, characterized in that it further comprises a tank cover (9) having a connector (9a) adapted to connect the interior of the tank (1) to the atmosphere.

6. Unit according to one or more of the preceding claims, characterized in that said wall of defoaming material (2) has a cylindrical shape and is divided into two parts which include a lower part (5) provided with said inlet connection (7) for blood coming from a patient and an upper part (8) provided with said inlet connection (10) for blood coming from an operating field, said upper part being upwardly delimited by a portion of said tank cover (9) in which said inlet connection (10) for blood coming from an operating field is located.

7. Unit according to claim 6, characterized in that said lower part (5) of said wall of defoaming material is provided with one filter (11) and said upper part (8) is also provided with said one filter (11) an additionally with a further filter (12).

8. Unit according to one or more of the preceding claims, characterized in that said tank cover (9) is rotatably connected to said tank (1) with the interposition therebetween of sealing gaskets (9b) for allowing said tank to be arranged in depression.

9. Unit according to one or more of the preceding claims, characterized in that it further comprises a bypass line (28) interconnected between the outlet connection of the oxygenator with one (7) of the inlet connections of the tank.

10. Unit according to one or more of the preceding claims, characterized in that said membrane (29) is provided with a tab (29a) adapted to be gripped by an operator to return said membrane from the occlusion position to the opening one, said mechanical safety device (26) further comprising a small cover (30) adapted to limit the elastic deformation of the membrane in condition of opening of the connection.

11. Unit according to one or more of the preceding claims, characterized in that said mechanical safety device (26) is arranged in an autonomous containment structure adapted to be connected to said tank.

## Patentansprüche

1. Eine integrierte Behandlungseinheit für die Verwendung in einem extrakorporalen Blutkreislauf, enthaltend:
- einen Behälter (1), der im Inneren mit einer Wand aus Entschäumungsmaterial (2) versehen ist und wenigstens einen Filter (11, 12) enthält;
- einen Einlaufanschluß (7) für das von einem Patienten kommende Blut und einen Einlaufanschluß (10) für das von einem Operationsfeld kommende Blut, wobei die Anschlüsse (7 und 10) derart angeordnet sind, daß das von ihnen kommende Blut durch das Entschäumungsmaterial (2) und den Filter (11, 12) gelangt, wonach das Blut in dem Behälter (1) gehalten wird, so daß der Behälter gleichzeitig als Cardiotome und Behälter für das venöse Blut wirkt; und
- einen am Boden des Behälters vorgesehenen Auslaufanschluß (1a) für den Ausfluß des in ihm enthaltenen Blutes,
wobei die integrierte Einheit durch folgende Merkmale gekennzeichnet ist:
- einen Wärmetauscher (15) und einen Oxygenator (14), die beide einstückig mit dem Behälter (1) verbunden und derart ausgebildet sind, daß sie von dem vom Auslaufanschluß (1a) des Behälters (1) ausfließenden Blut durchflossen werden; und
- eine mechanische Sicherheitsvorrichtung (26), die am Auslaufanschluß (1a) des Behälters vorgesehen ist, um das Schließen des Auslaufanschlusses bei Entleerung des Behälters zu bestimmen,
wobei die mechanische Sicherheitsvorrichtung eine elastische Membran (29) enthält, die derart ausgebildet ist, daß sie durch den hydrostatischen Druck des anliegenden Blutes in einer derartigen Stellung gehalten wird, daß der Auslaufanschluß offengehalten wird und daß sie sich zum Verschließen derselben bewegt, wenn der Druck einen Wert erreicht hat, der einem Minimalpegel des Blutes im Behälter entspricht.

2. Vorrichtung nach Anspruch 1, dadurch gekennzeichnet, daß sie weiterhin eine Blutzirkulationspumpe (25) enthält, deren Ansaugleitung mit dem Auslaufanschluß (1a) verbunden ist und deren Auslaß mit einem Bluteinlaufanschluß (22) des Wärmetauschers (15) verbunden ist, wobei der Wärmetauscher einen Auslaß aufweist, der mit dem Einlaß des Oxygenators (14) verbunden ist, von dem das Blut zum Patienten zurückgelangt.

3. Vorichtung nach Anspruch 1, dadurch gekennzeichnet, daß der Auslaufanschluß (1a) des Behälters (1) direkt mit einem Einlauf (22) des Wärmetauschers (15) verbunden ist und die Einheit weiterhin eine am Einlauf des Oxygenators (14) angeordnete Pumpe enthält, die zur Bestimmung der Blutzirkulation angepaßt ist und an ihrer Ansaugstelle mit einem Auslauf des Wärmetauschers (15) verbunden ist.

4. Vorrichtung nach einem oder mehreren der vorhergehenden Ansprüche, dadurch gekennzeichnet, daß der Behälter (1) in einer Stellung über einem einstückigen Gebilde angeordnet ist, das oben einen Oxygenator (14) und unten einen Wärmetauscher (15) aufweist und mit ihm über eine Schnellverbindung (13) verbunden ist.

5. Vorrichtung nach einem oder mehreren der vorhergehenden Ansprüche, gekennzeichnet durch einen Behälterdeckel (19) mit einem Anschluß (9a), der das Innere des Behälters (1) mit der Atmosphäre verbindet.

6. Vorrichtung nach einem oder mehreren der vorhergehenden Ansprüche, dadurch gekennzeichnet, daß die Wand des Entschäumungsmaterials (2) eine zylindrische Form hat und in zwei Teile geteilt ist, die einen unteren Teil (5) einschließen, der mit dem Einlaufanschluß (7) für das vom Patienten kommende Blut versehen ist und einen oberen Teil (8) einschließen, der mit dem Einlaufanschluß (10) für das von einem Operationsfeld kommende Blut versehen ist, wobei der obere Teil nach oben durch einen Teil des Behälterdeckels (9) begrenzt wird, in dem der Einlaufanschluß (10) für das vom Operationsfeld kommende Blut angeordnet ist.

7. Vorrichtung nach Anspruch 6, dadurch gekennzeichnet, daß der untere Teil (5) der Wand des Entschäumungsmaterials mit einem Filter (11) versehen ist und der obere Teil (8) ebenfalls mit diesem Filter (11) und zusätzlich mit einem weiteren Filter (12) versehen ist.

8. Vorrichtung nach einem oder mehreren der vorhergehenden Ansprüche, dadurch gekennzeichnet, daß der Behälterdeckel (9) drehbar mit dem Behälter (1) verbunden ist, wobei dazwischen Abdichtungen (9b) eingefügt sind, die es erlauben, den Behälter in Unterdruck zu versetzen.

9. Vorrichtung nach einem oder mehreren der vorhergehenden Ansprüche, dadurch gekennzeichnet, daß sie weiterhin eine Bypassleitung (28) aufweist, die zwischen dem Auslaufanschluß des Oxygenators und einem (7) der Einlaufanschlüsse des Behälters eingefügt ist.

10. Vorrichtung nach einem oder mehreren der vorhergehenden Ansprüche, dadurch gekennzeichnet, daß die Membran (29) mit einem Zapfen (29a) versehen ist, der von einer Bedienperson gegriffen werden kann, um die Membran von der Schließstellung in die Öffnungsstellung zurückzuführen, wobei die mechanische Sicherheitsvorrichtung (26) weiterhin eine kleine Abdeckung (30) aufweist, die die elastische Deformation der Membran im Zustand des geöffneten Anschlusses begrenzt.

11. Vorrichtung nach einem oder mehreren der vorhergehenden Ansprüche, dadurch gekennzeichnet, daß die mechanische Sicherheitsvorrichtung (26) in einer selbständigen Behälterstruktur angeordnet ist, die am Behälter befestigbar ist.

## Revendications

1. Unité intégrée pour une utilisation dans un circuit sanguin extracorporel, comprenant :
- un réservoir (1) intérieurement muni d'une paroi en matière anti-moussante (2) comportant au moins un filtre (11,12) ;
- une liaison d'entrée (7) pour le sang venant d'un patient et une liaison d'entrée (10) pour le sang venant d'un champ opératoire, lesdites liaisons (7 et 10) étant agencées pour faire passer le sang venant de ceux-ci à travers ladite matière anti-moussante (2) et le filtre (11,12), après quoi le sang est maintenu dans ledit réservoir (1) et ainsi ledit réservoir agit, simultanément, à la fois comme réservoir veineux et de cardiotomie ; et
- une liaison de sortie (1a) agencée au fond dudit réservoir pour l'écoulement du sang contenu dans celui-ci ;
l'unité intégrée étant caractérisée en ce qu'elle comprend, de plus :
- un échangeur de chaleur (15) et un oxygénateur (14) qui sont tous les deux reliés, de façon monolithique, audit réservoir (1) et qui sont adaptés pour être traversés par le sang s'écoutant de ladite liaison de sortie (1a) dudit réservoir (1) ; et
- un dispositif de sécurité mécanique (26) agencé sur ladite liaison de sortie (1a) dudit réservoir pour déterminer la fermeture de ladite liaison de sortie lors de la vidange du réservoir,
ledit dispositif de sécurité mécanique comprenant une membrane élastique (29) adaptée pour être maintenue, par la charge hydrostatique du sang la recouvrant, dans une configuration pour garder la liaison de sortie ouverte, et pour se déplacer pour fermer celle-ci quand ladite charge atteint une valeur correspondant à un niveau minimum de sang dans le réservoir.

2. Unité selon la revendication 1,
caractérisée en ce qu'elle comprend, de plus, une pompe de circulation sanguine (25) dont la ligne d'aspiration est reliée à ladite liaison de sortie (1a) et dont la ligne de refoulement est reliée à une liaison d'entrée du sang (22) dudit échangeur de chaleur (15), ledit échangeur de chaleur ayant une sortie reliée à une entrée de l'oxygénateur (14) à partir duquel le sang retourne au patient.

3. Unité selon la revendication 1,
caractérisée en ce que ladite liaison de sortie (1a) dudit réservoir (4) est directement reliée à une entrée (22) dudit échangeur de chaleur (15), l'unité comprenant de plus, agencée à une entrée dudit oxygénateur (14), une pompe adaptée pour déterminer la circulation du sang et reliée, à son aspiration, à une sortie dudit échangeur de chaleur (15).

4. Unité selon une ou plusieurs des revendications précédentes,
caractérisée en ce que ledit réservoir (1) est agencé dans une position recouvrant une structure monolithique comprenant en haut ledit oxygénateur (14) et en bas ledit échangeur de chaleur (15), et est relié à celle-ci au moyen d'une liaison rapide (13).

5. Unité selon une ou plusieurs des revendications précédentes,
caractérisée en ce qu'elle comprend, de plus, un couvercle (9) de réservoir, ayant un raccord (9a) adapté pour relier l'intérieur du réservoir (1) à l'atmosphère.

6. Unité selon une ou plusieurs des revendications précédentes,
caractérisée en ce que ladite paroi de matière anti-moussante (2) présente une forme cylindrique et est divisée en deux parties, qui comprennent une partie inférieure (5), munie de ladite liaison d'entrée (7) pour le sang venant du patient, et une partie supérieure (8), munie de ladite liaison d'entrée (10) pour le sang venant du champ opératoire, ladite partie supérieure étant délimitée en haut par une portion dudit couvercle (9) de réservoir, dans laquelle ladite liaison d'entrée (10) pour le sang venant du champ opératoire est située.

7. Unité selon la revendication 6,
caractérisée en ce que ladite partie inférieure (5) de ladite paroi de matière anti-moussante est munie d'un filtre (11), et ladite partie supérieure (8) est également munie dudit filtre (11) et, en plus, d'un autre filtre (12).

8. Unité selon une ou plusieurs des revendications précédentes,
caractérisée en ce que ledit couvercle (9) de réservoir est relié, de façon rotative, audit réservoir (1) avec l'inter-position entre eux de joints d'étanchéité (9b) pour permettre audit réservoir d'être agencé en dépression.

9. Unité selon une ou plusieurs des revendications précédentes,
caractérisée en ce qu'elle comprend, de plus, une ligne de dérivation (28) interposée entre la liaison de sortie de l'oxygénateur et une (7) des liaisons d'entrée du réservoir.

10. Unité selon une ou plusieurs des revendications précédentes,
caractérisée en ce que ladite membrane (29) est munie d'une patte (29a) agencée pour être saisie par un opérateur pour ramener ladite membrane de la position d'occlusion à la position d'ouverture, ledit dispositif de sécurité mécanique (26) comprenant, de plus, un petit couvercle (30) adapté pour limiter la déformation élastique de la membrane en condition d'ouverture de la liaison.

11. Unité selon une ou plusieurs des revendications précédentes,
caractérisée en ce ledit dispositif de sécurité mécanique (26) est agencé dans une structure de confinement autonome adaptée pour être reliée audit réservoir.
